# EUROPEAN PATENT APPLICATION

(11) **EP 3 144 829 A1**
(43) Date of publication of application: **22.03.2017**
(21) Application number: 16160159.6
(22) Date of filing: 14.03.2016
(51) Int. Cl.: G06F 17/30, G06F 19/00

(54) **MEDICAL-DOCUMENT MANAGEMENT APPARATUS, ELECTRONIC MEDICAL RECORD SYSTEM, MEDICAL-DOCUMENT MANAGEMENT SYSTEM, AND PROGRAM**

(30) Priority: 16.09.2015 JP 2015183010
(71) Applicant: Fuji Xerox Co., Ltd., Minato-ku, Tokyo (JP)
(72) Inventor: TOSHIMITSU, Koki, Yokohama-shi, Kanagawa (JP)
(74) Representative: Ward, James Norman

(57) **Abstract**

A medical-document management apparatus includes a controller displaying, in an arranged manner, a matrix information display section in which, by accessing a database, medical documents registered in the database are subjected to two-dimensional mapping on date and attribute and are displayed; a document-to-be-registered operating section in which a first medical document to be registered in the database is set; an associated-form setting section in which a second medical document among the registered medical documents which is associated with the first medical document is set; and a document attribute setting section in which attributes of the first medical document are set, on a screen of a terminal apparatus. The controller also records, in the database, link information which describes a link between the first and second medical documents and which is set in the associated-form setting section.

## Description

### BACKGROUND

### (i) Technical Field

The present invention relates to a medical-document management apparatus, an electronic medical record system, a medical-document management system, and a program.

### (ii) Related Art

In recent community medicine, attention is being focused on systems in which various medical institutions or organizations disclose medical information in order to share patient information created in the institutions.

JP-A-2015-28680 discloses a technique for obtaining time and attributes (such as a diagnostic site, treatment information, and a document) of each piece of medical data, mapping the obtained data on the same time series (X axis) and attribute items (Y axis), and displaying the mapped data for displaying medical data provided from a plurality of hospitals on one browser screen collectively.

### SUMMARY

It is effective that time and attributes of medical data provided from a plurality of hospitals are obtained, and mapped on the same time series (X axis) and attribute items (Y axis), and displayed, but searching of medical data associated with specific medical data is not sufficiently examined. Accordingly, every time medical data associated with certain medical data is searched, searching and displaying operations are required to be performed repeatedly, and time for accessing the associated medical data is required. Furthermore, when there is a huge amount of information, it cannot be accessed to the associated medical data in some cases. In addition, in a case where when mapping data on the same time series (X axis) and attribute items (Y axis), a plurality of documents is recorded in one cell, searching of a document under the case is not sufficiently examined, and searching and displaying operations are required to be performed repeatedly for confirming document details. For searching diagnostic data associated with certain diagnostic data, both diagnostic data pieces are required to be associated with each other. However, when it is difficult to process association, operability is degraded. As a result, searching cannot be effectively performed.

An object of the invention is to provide a medical-document management apparatus, a medical-document management system, and a program which are capable of performing searching of medical data associated with specific medical data more easily, in a case where time and attributes of medical data provided from a plurality of hospitals are obtained, and mapped two-dimensionally on the same time series (X axis) and attribute items (Y axis), and displayed.
[1] According to an aspect of the invention, there is provided a medical-document management apparatus including a controller. The controller displays, in an arranged manner, a matrix information display section, a document-to-be-registered operating section, an associated-form setting section, and a document attribute setting section on a screen of a terminal apparatus. The matrix information display section is a section in which, by accessing a database, registered medical documents registered in the database are subjected to two-dimensional mapping on date and attribute and are displayed. The document-to-be-registered operating section is a section in which a first medical document to be registered in the database is set. The associated-form setting section is a section in which a second medical document which is among the registered medical documents and which is associated with the first medical document is set. The document attribute setting section is a section in which attributes of the first medical document are set. The controller records, in the database, link information describing a link between the first medical document and the second medical document. The link information is set in the associated-form setting section.
[2] In the medical-document management apparatus according to [1], the controller may further display, in an arranged manner, an associated-form setting palette section on the screen of the terminal apparatus, and, when a specific medical document among the medical documents which are mapped and displayed in the matrix information display section is moved to the associated-form setting palette section, the controller displays the specific medical document in the associated-form setting palette section, and displays the specific medical document in the associated-form setting section in a list format.
[3] In the medical-document management apparatus according to [1], the controller may use an operation of viewing a medical document registered in the database, as a trigger to display, in an arranged manner, the matrix information display section, the document-to-be-registered operating section, the associated-form setting section, and the document attribute setting section on the screen of the terminal apparatus, the viewing operation being performed in creation of an electronic medical record on the terminal apparatus.
[4] According to another aspect of the invention, there is provided a medical-document management system including a database; a terminal apparatus; and a medical-document management apparatus according to [1]. In response to a request from the terminal apparatus, the controller may display, in an arranged manner, the matrix information display section, the document-to-be-registered operating section, the associated-form setting section, and the document attribute setting section on a screen of the terminal apparatus, and records, in the database, link information describing a link between the first medical document and the second medical document, the link information being set in the associated-form setting section.
[5] According to another aspect of the invention, there is provided a program causing a computer to execute a process including: by accessing a database, subjecting registered medical documents to two-dimensional mapping on date and attribute and displaying the mapped data on a terminal apparatus, the registered medical documents being registered in the database; displaying a document-to-be-registered operating section on the terminal apparatus in an arranged manner, the document-to-be-registered operating section being a section in which a first medical document to be registered in the database is set; displaying an associated-form setting section on the terminal apparatus in an arranged manner, the associated-form setting section being a section in which a second medical document which is among the registered medical documents and which is associated with the first medical document is set; displaying a document attribute setting section on the terminal apparatus in an arranged manner, the document attribute setting section being a section in which attributes of the first medical document are set; and recording, in the database, link information describing a link between the first medical document and the second medical document, the link information being set in the associated-form setting section.

According to the medical-document management apparatus of [1], the medical-document management system of [4], and the program of [5], the searching of the medical data associated with specific medical data can be more easily performed than that of the related art.

According to the medical-document management apparatus of [2], the association required for searching can be more easily performed.

According to the medical-document management apparatus of [3], the association required for searching can be more easily performed in cooperation with the creation of the electronic medical record.

### BRIEF DESCRIPTION OF DRAWINGS

Exemplary embodiments of the present invention will be described in detail based on the following figures, wherein:
Fig. 1 is a diagram illustrating a system configuration according to an exemplary embodiment;
Fig. 2 is a diagram for describing an exemplary terminal screen according to the exemplary embodiment;
Fig. 3 is a diagram for describing a matrix information display section according to the exemplary embodiment;
Fig. 4 is a diagram for describing an associated-form setting section according to the exemplary embodiment;
Fig. 5 is a diagram for describing an associated-form setting palette section according to the exemplary embodiment;
Fig. 6 is a diagram for describing a document-to-be-registered operating section according to the exemplary embodiment; and
Fig. 7 is a diagram for describing a document attribute setting section according to the exemplary embodiment.

### DETAILED DESCRIPTION

Exemplary embodiments of the present invention will be described below on the basis of the drawings.

### Basic Principles

First, basic principles of the exemplary embodiments will be described.

In the exemplary embodiments, when a doctor in a certain hospital creates a medical document such as a medical record for a certain patient by referring to other medical documents which have been already created for the patient, link information in which the medical documents which have been referred to are set as a link target is attached to the created medical document. When the created medical document is recorded in a database or the like, the link information is also recorded in the database along with the medical document.

For example, a certain patient takes a medical examination in A hospital, and a doctor in A hospital creates a medical document A. In the case where the patient is transferred from A hospital to B hospital for some reason, when a doctor in B hospital creates a medical document such as a medical record, the doctor typically refers to the medical document which has been created in A hospital from which the patient has been transferred, so as to obtain necessary information about the patient.

Therefore, in the exemplary embodiments, in the case where, in creation of a medical document, a doctor refers to different medical documents, link information indicating that the different medical documents are viewed is created, and is attached to the medical document. The medical document created by the doctor in B hospital is referred to as a first medical document, and the existing registered medical document that is created by the doctor in A hospital and that is referred to when the first medical document is created is referred to as a second medical document. The first medical document and the second medical document are associated with each other by using the link information of the first medical document, and the second medical document is an associated document of the first medical document.

After that, similarly, every time a different medical document is referred to in creation of a medical document, link information is created in sequence, and is accumulated in the database or the like. As in the related art, a user may easily search for medical documents associated with a certain medical document on a two-dimensional map with time and attribute, for example, by giving emphasis indication to the associated medical documents of the certain medical document in display of medical information on the two-dimensional map. The term "emphasis indication" means that the associated medical documents are displayed so as to be easily and visually recognized relatively in comparison with the other medical documents (medical documents which are not associated with the certain medical document). Any way to show emphasis indication may be employed. Examples of emphasis indication include giving emphasis indication by using the same color or in the same pattern, giving emphasis indication in increased brightness, displaying a list in a different window, and giving emphasis indication by using a pop-up menu. Examples of emphasis indication may also include a practice in which any mark (for example, an arrow, a line, or a dashed line) indicating association between the first medical document and the second medical document is displayed when the first medical document is associated with the second medical document.

In creation of link information, an operation screen for setting medical documents as a link target is displayed on a terminal apparatus operated by a doctor. On the operation screen, a screen for setting a medical document which is to be newly registered in the database, a screen for setting medical documents associated with the medical document to be registered or with a different medical document, and a screen for setting attributes of a medical document are listed along with the two-dimensional map. By using such an operation screen, a doctor operating a terminal apparatus may easily associate medical documents with each other, and may record the association between the medical documents in the database.

### Configuration

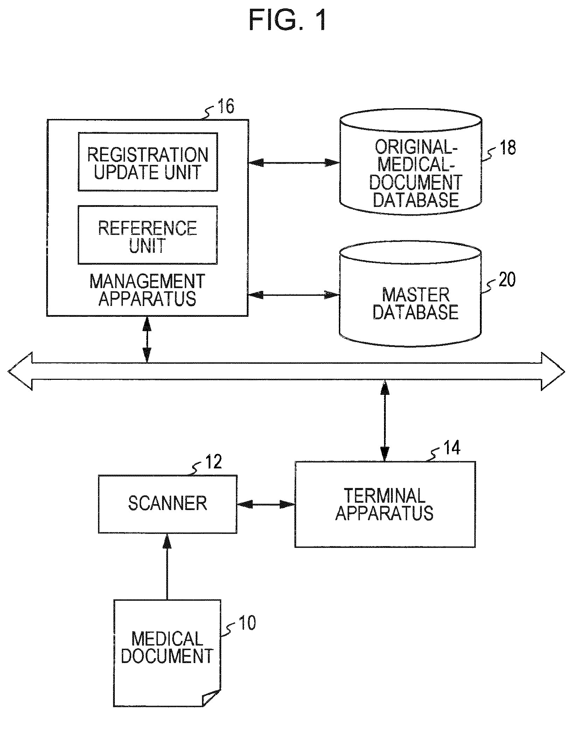

Fig. 1 is a diagram illustrating the system configuration of a medical-document management system according an exemplary embodiment. The medical-document management system includes a scanner 12, a terminal apparatus 14, a management apparatus 16, an original-medical-document database 18, and a master database 20, and the terminal apparatus 14 and the management apparatus 16 are connected to a network. The management apparatus 16 and the terminal apparatus 14 serve as a server computer and a client computer, respectively, in a server-client system. In Fig. 1, the original-medical-document database 18 and the master database 20 are not connected to the network. As a matter of course, the original-medical-document database 18 and the master database 20 may be connected to the network so as to be allowed to be accessed from other apparatuses.

The scanner 12 scans a medical document 10 and converts the medical document into an electronic format under the control of the terminal apparatus 14. The medical document 10 is typically a medical record created by a doctor. Other than this, any documents about medical care and diagnosis/treatment, such as a referral form, a consent form, and a notice document, may be included. Needless to say, a doctor may directly create a medical record (electronic medical record) by operating the terminal apparatus 14. The scanner 12 is desirably provided with a reading function of obtaining the creation time (date) and attributes (such as the patient name, a diagnostic site, and treatment information) of a medical document. When the date and the attributes are converted into a bar code which is printed on the medical document, the scanner 12 reads the bar code, and obtains the date and the attributes.

The terminal apparatus 14 transmits the medical document in an electronic format to the management apparatus 16. In addition, when a doctor refers to specific medical documents in creation of medical information, the terminal apparatus 14 transmits the reference information attached to the medical document as a link, to the management apparatus 16. The link is attached by the doctor operating the terminal apparatus 14. The operation screen on the terminal apparatus 14 will be described below. Thus, for example, when a doctor refers to a medical document B in creation of a medical document A, the following link is created:
medical document A → medical document B,
where an arrow (→) indicates a link. The number of links is not necessarily one, and may be more than one. For example, when the medical document B and a medical document C are referred to in creation of the medical document A, the following links are created:
   medical document A → medical document B
   medical document A → medical document C

When both of the medical document B and the medical document C are created by referring to a medical document D, the following links are created:
medical document A → medical document B → medical document D
medical document A → medical document C → medical document D
In this case, medical documents associated with the medical document A are the medical document B, the medical document C, and the medical document D.

The terminal apparatus 14 transmits a view request along with an ID for specifying the terminal apparatus 14, to the management apparatus 16 in order to view medical documents recorded in the original-medical-document database 18.

The management apparatus 16 records a medical document in an electronic format in the original-medical-document database 18. In addition, in response to a view request from the terminal apparatus 14, the management apparatus 16 accesses the original-medical-document database 18 to obtain medical documents, and returns the medical documents to the terminal apparatus 14. In the original-medical-document database 18, medical documents created in multiple hospitals are recorded.

The management apparatus 16 records information about a medical document in an electronic format, specifically, the date and attributes (such as patient information, document type information, and institution information), user information, and link information of the medical document, in the master database 20. The institution information is information about a hospital or a clinic. The user information is information about a hospital and a doctor using the system. The management apparatus 16 obtains the dates and the attributes of medical documents from the master database 20, maps the medical documents on the same time series (X axis) and attribute items (Y axis), and displays the mapped data. In addition, if link information is present for a medical document, the management apparatus 16 gives emphasis indication to the associated medical documents in accordance with the link information.

The management apparatus 16 includes a medical-document receiving/transmitting unit, a medical-document recording unit, a master data recording unit, and a medical-document editing/displaying unit as functional blocks. Specifically, the management apparatus 16 is constituted by a computer (server computer), and includes a central processing unit (CPU), a program memory, a working memory, and an input/output interface. The CPU executes various types of processing according to processing programs recorded in the program memory. Among the above-described functional blocks, the medical-document receiving/transmitting unit is a unit which is not illustrated in Fig. 1 and for which most of the functions are provided by the management apparatus 16; the medical-document recording unit corresponds to the original-medical-document database 18 illustrated in Fig. 1; most part of the master data recording unit corresponds to the master database 20 illustrated in Fig. 1; and most part of the medical-document editing/displaying unit corresponds to a registration update unit and a reference unit which are included in the management apparatus 16 in Fig. 1. Processes performed by the CPU of the management apparatus 16 are as follows:
▪ Displaying the operation screen for generating link information, on the terminal apparatus 14
▪ Receiving a medical document and link information from the terminal apparatus 14
▪ Transmitting the received medical document to the original-medical-document database 18 to record the medical document, associating the date, the attributes, and the link information of the medical document with each other, and transmitting the associated information to the master database 20 to record the associated information
· Receiving a view request from the terminal apparatus 14
· In response to the view request from the terminal apparatus 14, reading medical documents from the original-medical-document database 18, editing the medical documents, and displaying the edited data on the terminal apparatus 14

The original-medical-document database 18 records medical documents created in multiple hospitals, in sequence.

The master database 20 records, in sequence, the user information, the patient information, the document type information, the institution information, and the link information which are associated with a medical document.

Table 1 illustrates exemplary institution information stored in the master database 20, in a table format.

**Table 1**

| Hospital ID | Hospital name | ··· |
|---|---|---|
| A | A hospital | |
| B | B hospital | |
| C | C hospital | |
| D | D hospital | |

Table 2 illustrates exemplary link information stored in the master database 20, in a table format.

**Table 2**

| Document ID | Hospital ID | Document name | Document type name | Associated form |
|---|---|---|---|---|
| docB | B | test report B | doctypeB | docC |
| | | | | docD |
| docC | C | treatment report C | doctypeC | docB |
| | | | | docD |
| docD | D | medical-examination report D | doctypeD | docC |

Table 2 indicates that the medical document "test report B" is created in B hospital; the document ID of the medical document is docB; and docC and docD are set as a link target. Table 2 also indicates that the medical document "treatment report C" is created in C hospital; the document ID of the medical document is docC; and docB and docD are set as a link target.

In Fig. 1, the original-medical-document database 18 and the master database 20 are illustrated as separate databases. Alternatively, the original-medical-document database 18 and the master database 20 may constitute a single database recording medical documents.

The operation screen displayed on the screen of the terminal apparatus 14 by the management apparatus 16 in order to create link information will be described.

### Operation Screen

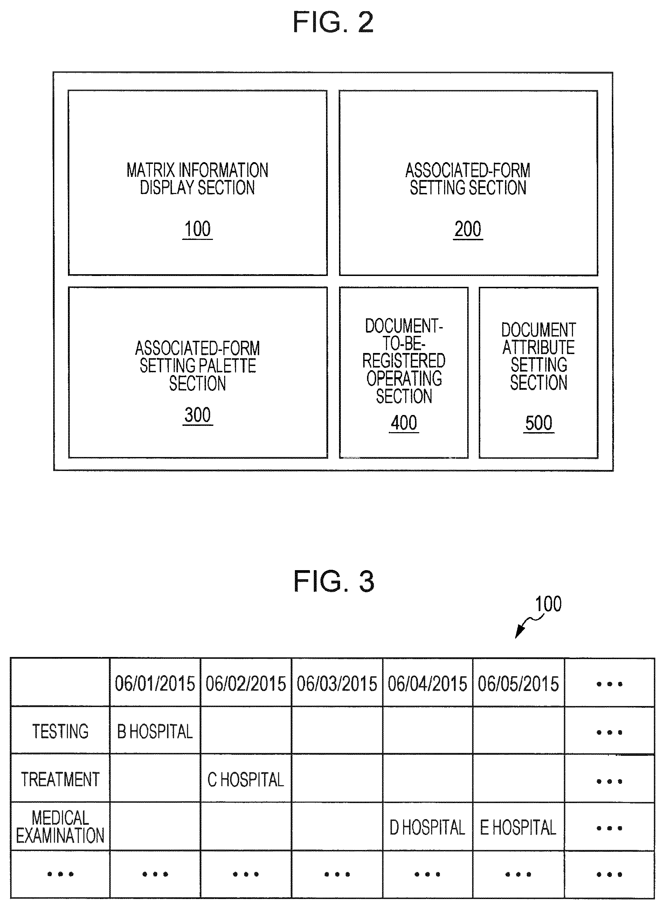

Fig. 2 illustrates an operation screen displayed by the management apparatus 16 on the screen of the terminal apparatus 14 which has accessed the management apparatus 16.

A matrix information display section 100 and an associated-form setting section 200 are displayed in the upper row of the screen on the terminal apparatus 14. An associated-form setting palette section 300, a document-to-be-registered operating section 400, and a document attribute setting section 500 are displayed in the lower row of the screen.

The matrix information display section 100 is displayed by obtaining the dates and the attributes of medical documents recorded in the original-medical-document database 18, and mapping the icons for the medical documents on the same time series (X axis) and attribute items (Y axis).

In the associated-form setting section 200, a list of the medical documents disposed in the associated-form setting palette section 300 is displayed. The listed medical documents may include a medical document created in the hospital in which the operation screen is displayed, and a medical document created in another hospital. In the associated-form setting section 200, associated medical documents related to the hospital in which the operation screen is displayed may be added or deleted.

A drag-and-drop operation is performed on the icon for a medical document included in one cell in the matrix information display section 100, whereby the medical document and its linked medical documents are displayed in an arranged manner in the associated-form setting palette section 300.

The document-to-be-registered operating section 400 is a display section for operating the scanner 12 to convert the medical document 10 into an electronic format and obtain the data.

The document attribute setting section 500 is a display section for setting attributes and link information of a medical document and recording the attributes and the link information in the original-medical-document database 18 and the master database 20 via the management apparatus 16.

Each of these display sections will be described below.

Fig. 3 illustrates the detail of the matrix information display section 100. The icons for medical documents created in multiple hospitals are mapped in the matrix information display section 100 in which X axis represents date, and Y axis represents attributes (such as (testing, treatment, and medical examination). For example, the icon for a medical document created in B hospital is mapped for the date 06/01/2015 and the testing, and the icon for a medical document created in C hospital is mapped for the date 06/02/2015 and the treatment. Any icon form for a medical document may be employed. A document icon is typically employed, but this is not limiting. When multiple medical documents are present, multiple document icons may be displayed in a superimposed manner. A user may drag and drop the icon for a medical document which is included in one cell in the matrix information display section 100, to the associated-form setting palette section 300.

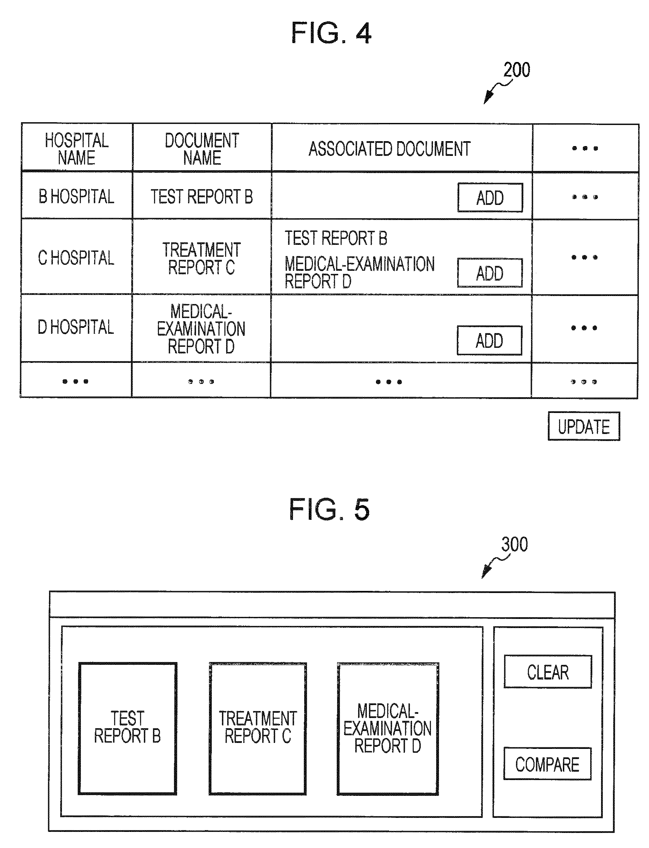

Fig. 4 illustrates the detail of the associated-form setting section 200. In the associated-form setting section 200, a list of medical documents disposed in the associated-form setting palette section 300 is displayed. Fig. 4 illustrates a case in which the medical documents disposed in the associated-form setting palette section 300 are a test report B created in B hospital, a treatment report C created in C hospital, and a medical-examination report D created in D hospital. A list of these medical documents is displayed. When new link information is to be set to these medical documents, a medical document is selected from the medical documents displayed in the associated-form setting palette section 300, and an add button is operated. In Fig. 4, the test report B and the medical-examination report D are already set as link information for the treatment report C, and these documents are displayed in a list format. In addition or deletion of link information, when an update button is operated, link information obtained after the addition or deletion is recorded in the master database 20 via the management apparatus 16.

Fig. 5 illustrates the detail of the associated-form setting palette section 300. In Fig. 5, medical documents, i.e., the test report B, the treatment report C, and the medical-examination report D, are displayed in an arranged manner. This is obtained by dragging and dropping the icon for the medical document created in C hospital in the matrix information display section 100. The medical document created in C hospital, i.e., the treatment report C, is displayed in the associated-form setting palette section 300, and the associated documents, i.e., the test report B and the medical-examination report D which are set as link information for the treatment report C, are displayed in an arranged manner.

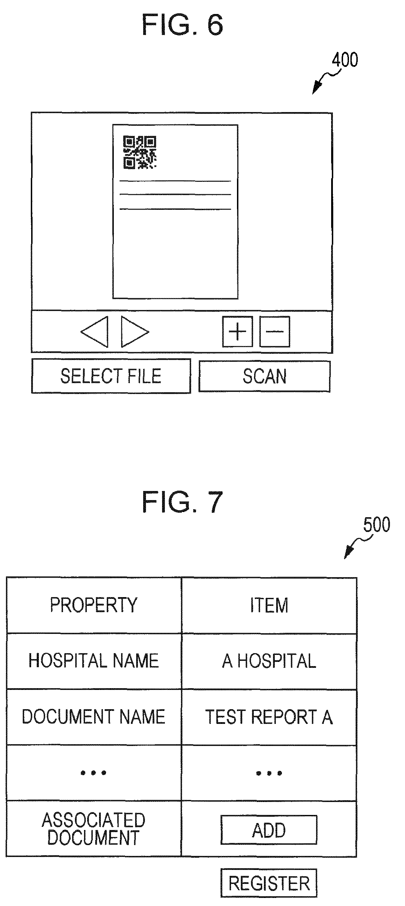

Fig. 6 illustrates the detail of the document-to-be-registered operating section 400. In the document-to-be-registered operating section 400, a medical document that is to be newly recorded in the original-medical-document database 18 is specified by dragging and dropping the medical document in a predetermined frame. As described above, the scanner 12 may be operated so that the medical document is converted into an electronic format so as to be obtained. Fig. 6 illustrates a case in which a medical document having a QR code^{®} in which a date and attributes are written is obtained by using the scanner 12.

In the document-to-be-registered operating section 400, in addition to a medical document to be newly registered, medical documents associated with the medical document may be displayed. Thus, in the document attribute setting section 500, not only the attributes of the medical document to be newly registered, but also the attributes of the medical documents associated with the medical document may be set at the same time.

Fig. 7 illustrates the detail of the document attribute setting section 500. The attributes, i.e., properties and items, of a medical document dragged and dropped to the document-to-be-registered operating section 400, or of a medical document obtained by using the scanner 12 are set. The properties are the hospital name, the document name, associated documents, and the like of a medical document obtained in the document-to-be-registered operating section 400. Fig. 7 illustrates a case in which "A hospital" is input as the hospital name; and "test report A" is input as the document name. If associated medical documents are present, the attributes of the associated medical documents are similarly set. After attributes are set, a registration button is operated, whereby the medical document, the attributes, and the link information are recorded in the original-medical-document database 18 and the master database 20 via the management apparatus 16.

A series of operations on the operation screen will be described below.

A user, i.e., a doctor in a hospital, operates the terminal apparatus 14 so as to access the management apparatus 16, and requests medical documents to be viewed.

When the management apparatus 16 receives a view request from the terminal apparatus 14, the management apparatus 16 accesses the original-medical-document database 18 and the master database 20, obtains medical documents, the attributes, and the link information, creates screen data including the matrix information display section 100 in which the icons for the medical documents are mapped, transmits the created screen data to the terminal apparatus 14, and displays the data on the screen of the terminal apparatus 14.

The user checks the matrix information display section 100 displayed on the screen of the terminal apparatus 14. If a medical document to be referred to is present, the user selects the icon for the medical document. When the icon is selected, the selected medical document is displayed, and the user may check the information in the medical document.

When the user refers to the medical document, the user drags and drops the icon for the medical document to the associated-form setting palette section 300. Thus, in the associated-form setting palette section 300, the selected medical document and its associated medical documents are displayed in an arranged manner. The associated medical documents are specified by using the link information of the selected medical document. A duplicate medical document is deleted, and medical documents are then displayed in an arranged manner.

All of the medical documents displayed in an arranged manner in the associated-form setting palette section 300 are automatically displayed in the associated-form setting section 200 as a list. The user may select a medical document from the medical documents displayed in the associated-form setting palette section 300, and may add the selected medical document as an associated document of a medical document displayed in the associated-form setting section 200. For example, in the example in Fig. 4, no associated information is present for the test report B created in B hospital. The treatment report C may be selected and may be added as an associated document of the test report B by using the add button.

A user drags and drops a medical document which is newly created, or converts the document into an electronic format by using the scanner 12 so as to obtain the medical document, thereby displaying the medical document to be newly registered, in the document-to-be-registered operating section 400. If associated documents of the medical document are present, the associated documents are displayed similarly through drag-and-drop operations. The user operates the document attribute setting section 500 so as to set the attributes of the medical document to be newly registered, and the attributes of the associated documents, and operates the registration button.

In response to the operation using the registration button on the terminal apparatus 14, the management apparatus 16 records the medical document in the original-medical-document database 18, and creates link information describing links between the medical document and the associated documents, and records the link information in the master database 20. The link information is described as the associated form in Table 2.

Thus, in the exemplary embodiment, a screen as illustrated in Fig. 2 is displayed on the terminal apparatus 14, whereby medical documents associated with a certain medical document may be easily set and may be recorded in the original-medical-document database 18 and the master database 20.

Thus, in the exemplary embodiment, by using the operation screen illustrated in Fig. 2, the following operations may be performed:
(1) Registering a new medical document in the database in such a manner that link information describing a link to a medical document which has been already registered in the database is attached to the new medical document
(2) Updating a medical document which has been already registered in the database, with link information that is attached to the medical document and that describes a link to another medical document which has been already registered in the database
(3) Performing a registration/update operation, for example, in which a medical document which has been already registered in the database is updated by deleting link information of the medical document

The exemplary embodiments are described above. These are not limiting, and various changes may be made.

For example, the system according to the exemplary embodiment may operate with an electronic medical record system in a linked manner. In creation of an electronic medical record in the electronic medical record system, the management apparatus 16 according to the exemplary embodiment may display the operation screen illustrated in Fig. 2 on the terminal apparatus 14 at an appropriate timing. In creation of an electronic medical record, a doctor often appropriately refers to a medical document which has been already created. Therefore, such a linked system is suitable for actual practices done by a doctor. For example, when a different medical document is referred to in input of an electronic medical record, a screen illustrated in Fig. 2 may be automatically displayed on the terminal apparatus 14, and the different medical document which is referred to may be automatically displayed in the associated-form setting palette section 300. More specifically, when a medical document recorded in the original-medical-document database 18 is viewed in input of an electronic medical record on the terminal apparatus 14, the management apparatus 16 displays the operation screen illustrated in Fig. 2 on the terminal apparatus 14 by using the view operation as a trigger, and prompts a user to set association with the viewed medical document.

In the exemplary embodiment, a table as illustrated as Table 2 is used to record attributes and link information of a medical document in the master database 20. Other than this, a flag which is set to describe whether or not a medical document is to be published may be recorded. In this case, a medical document not to be published is not allowed to be viewed, and is not registered as an associated document.

Table 3 illustrates, in a table format, exemplary link information recorded in the master database 20 in the case where institutions to which a medical document is to be published are specified.

**Table 3**

| Document ID | Hospital ID | Document name | Document type name | Associated form | Where to be published |
|---|---|---|---|---|---|
| docB | B | test report B | doctypeB | docC | all |
| | | | | docD | |
| docC | C | treatment report C | doctypeC | docB | D |
| | | | | docD | |
| docD | D | medical-examination report D | doctypeD | docC | not to be published |

Table 3 indicates that all of the hospitals are specified as institutions to which the medical document docB is to be published; only D hospital is specified as an institution to which the medical document docC is to be published; and the medical document docD is specified as being not to be published.

The foregoing description of the exemplary embodiments of the present invention has been provided for the purposes of illustration and description. It is not intended to be exhaustive or to limit the invention to the precise forms disclosed. Obviously, many modifications and variations will be apparent to practitioners skilled in the art. The embodiments were chosen and described in order to best explain the principles of the invention and its practical applications, thereby enabling others skilled in the art to understand the invention for various embodiments and with the various modifications as are suited to the particular use contemplated. It is intended that the scope of the invention be defined by the following claims and their equivalents.

The description of embodiments may disclose the following matters.
[1] It is an electronic medical record system including: an input section that receives information about medical examination for a patient; and a matrix information display section that is a section in which registered medical documents registered in a database are subjected to two-dimensional mapping on date and attribute and are displayed, wherein, when a view operation is performed on the registered medical documents on the matrix information display section, a document-to-be-registered operating section, an associated-form setting section, and a document attribute setting section are further displayed in an arranged manner, the document-to-be-registered operating section being a section in which a first medical document to be registered in the database is set, the associated-form setting section being a section in which a second medical document which is among the registered medical documents and which is associated with the first medical document is set, the document attribute setting section being a section in which attributes of the first medical document are set.

According to the electronic medical record system of the above [1], the searching of the medical data associated with specific medical data can be more easily performed than that of the related art.

## Claims

1. A medical-document management apparatus comprising:
a controller that displays, in an arranged manner, a matrix information display section, a document-to-be-registered operating section, an associated-form setting section, and a document attribute setting section on a screen of a terminal apparatus, the matrix information display section being a section in which, by accessing a database, registered medical documents registered in the database are subjected to two-dimensional mapping on date and attribute and are displayed, the document-to-be-registered operating section being a section in which a first medical document to be registered in the database is set, the associated-form setting section being a section in which a second medical document which is among the registered medical documents and which is associated with the first medical document is set, the document attribute setting section being a section in which attributes of the first medical document are set, and that records, in the database, link information describing a link between the first medical document and the second medical document, the link information being set in the associated-form setting section.

2. The medical-document management apparatus according to Claim 1,
wherein the controller further displays, in an arranged manner, an associated-form setting palette section on the screen of the terminal apparatus, and
wherein, when a specific medical document among the medical documents which are mapped and displayed in the matrix information display section is moved to the associated-form setting palette section, the controller displays the specific medical document in the associated-form setting palette section, and displays the specific medical document in the associated-form setting section in a list format.

3. The medical-document management apparatus according to Claim 1,
wherein the controller uses an operation of viewing a medical document registered in the database, as a trigger to display, in an arranged manner, the matrix information display section, the document-to-be-registered operating section, the associated-form setting section, and the document attribute setting section on the screen of the terminal apparatus, the viewing operation being performed in creation of an electronic medical record on the terminal apparatus.

4. A medical-document management system comprising:
a database;
a terminal apparatus; and
a medical-document management apparatus according to Claim 1,
wherein, in response to a request from the terminal apparatus, the controller displays, in an arranged manner, the matrix information display section, the document-to-be-registered operating section, the associated-form setting section, and the document attribute setting section on a screen of the terminal apparatus, and records, in the database, link information describing a link between the first medical document and the second medical document, the link information being set in the associated-form setting section.

5. A program causing a computer to execute a process comprising:
by accessing a database, subjecting registered medical documents to two-dimensional mapping on date and attribute and displaying the mapped data on a terminal apparatus, the registered medical documents being registered in the database;
displaying a document-to-be-registered operating section on the terminal apparatus in an arranged manner, the document-to-be-registered operating section being a section in which a first medical document to be registered in the database is set;
displaying an associated-form setting section on the terminal apparatus in an arranged manner, the associated-form setting section being a section in which a second medical document which is among the registered medical documents and which is associated with the first medical document is set;
displaying a document attribute setting section on the terminal apparatus in an arranged manner, the document attribute setting section being a section in which attributes of the first medical document are set; and
recording, in the database, link information describing a link between the first medical document and the second medical document, the link information being set in the associated-form setting section.
